# EUROPEAN PATENT APPLICATION

(11) **EP 1 552 851 A1**
(43) Date of publication of application: **13.07.2005**
(21) Application number: 03736068.2
(22) Date of filing: 06.06.2003
(51) Int. Cl.: A61K 47/12, A61K 9/20, A61K 31/4515, A61K 47/26, A61K 47/32, A61K 47/38, A61P 37/08

(54) **RAPIDLY DISINTEGRATING TABLET AND PROCESS FOR PRODUCING THE SAME**

(30) Priority: 10.06.2002 JP 2002168273
(71) Applicant: Dainippon Pharmaceutical Co., Ltd., Osaka-shi, Osaka 541-8524 (JP)
(72) Inventor: OGASAWARA, Kazuyoshi, Ikoma-gun, Nara 636-0144 (JP); SOGO, Kiyomi, Osaka-shi, Osaka 553-0001 (JP); YAMAMURA, Tadashi, Akashi-shi, Hyogo 673-0841 (JP)
(74) Representative: Coleiro, Raymond
(86) International application number: PCT/JP2003/007184
(87) International publication number: WO 2003/103713

(57) **Abstract**

This invention provides small-sized, intrabuccally rapidly disintegrating tablets comprising D-mannitol having an average particle diameter of 31 µm to 80 µm, an active ingredient, a disintegrant and stearic acid or a metallic stearate of 0.01 % by weight to 0.5 % by weight, which have sufficient hardness, do not disintegrate during usual handling, and have a good disintegrability in the oral cavity and a pleasant feeling in a mouth when administered, and further provides a method for producing an intrabuccally rapidly disintegrating tablet containing stearic acid or a metallic stearate in an amount of 0.01 % by weight to 0.5 % by weight, which comprises consecutively tableting a powder material containing D-mannitol having an average particle diameter of 31 µm to 80 µm, an active ingredient and a disintegrant by an external lubricating compression method, during which stearic acid or a metallic stearate as a lubricant is previously sprayed to adhere onto the punches and the dies of the tableting machine and the unadherent surplus of stearic acid or a metallic stearate is recovered.

## Description

### TECHNICAL FIELD

The present invention relates to a rapidly disintegrating tablet. More particularly, it relates to a tablet being rapidly disintegrated in the oral cavity (hereinafter, referred to as "intrabuccally rapidly disintegrating tablet") and a method for producing said intrabuccally rapidly disintegrating tablet by an external lubricating compression method.

### BACKGROUND ART

With the increase in the population of aged people, it has been desired to provide a pharmaceutical preparation which can easily be taken by aged people, but many of available pharmaceutical preparations for oral administration are in the conventional form of tablets or capsules, which is not necessarily easy to take for aged people. Besides, these conventional dosage forms are often difficult to be taken by children or patients having poor swallowing capability. Furthermore, powders or granules have also defects, for example, they need extra attention when they are unpacked, or they adhere to the oral cavity when they are taken, and hence they are not satisfactory enough for aged people, children or patients having poor swallowing capability. In order to solve these problems, some studies have already been done for seeking tablets that are easily taken without water and are easily handled.

WO 97/47287 discloses a method of producing a tablet which rapidly disintegrates in the oral cavity by means of a combination of a sugar alcohol or sugar having an average particle diameter of not more than 30 µm, an active ingredient and a disintegrant. This publication discloses that said method cannot give a product having a sufficient mechanical strength in case of using a sugar alcohol having a large average particle diameter as used in the present invention as mentioned hereinafter even if the tableting is carried out under a high compressing pressure. Furthermore, said publication also discloses another tableting method wherein a lubricant is previously coated onto the punches and the dies of the tableting machine before subjecting to compression, but it does not specifically disclose a method of producing tablets in a large scale, such as consecutively compressing while spraying a lubricant.

JP-A-2001-58944 discloses a rapidly disintegrable solid preparation, which comprises an active ingredient, D-mannitol having an average particle diameter of 30 µm to 300 µm, a disintegrant, and a cellulose. This publication discloses that a combination of an active ingredient with a comparatively coarse-grained sugar or sugar alcohol, a disintegrant and a cellulose can give an intrabuccally rapidly disintegrating tablet having a practically sufficient hardness and rapid-disintegration properties of the product even by compressing under a low pressure in dry system without problem in manufacturing thereof. However, said preparation is distinguished from the intrabuccally rapidly disintegrating tablet of the present invention explained below in that a combination of a cellulose and a disintegrant is used as an insoluble ingredient in said preparation.

JP-A-2000-103731 and JP-A-2000-344660 disclose a rapidly disintegrating solid preparation which comprises an active ingredient, a sugar and a low-substituted hydroxypropyl cellulose having a hydroxypropoxyl content of 5 % by weight to less than 7 % by weight, and further disclose that the preparation has insufficient solubility in the oral cavity when it is prepared by using a low-substituted hydroxypropyl cellulose having a hydroxypropoxyl content of 7 % by weight or more.

JP-A-2001-322927 discloses a solid preparation which comprises a low-substituted hydroxypropyl cellulose having an average particle diameter by volume of not more than 25 µm, measured by a laser diffraction analyzer in dry dispersion system, a loose bulk density of not less than 0.35 g/ml, and a tapped bulk density of not less than 0.60 g/ml, and a sugar or a sugar alcohol. And JP-A-2001-328948 discloses a solid preparation which comprises a low-substituted hydroxypropyl cellulose having a loose bulk density of not less than 0.40 g/ml and a tapped bulk density of not less than 0.60 g/ml, and a sugar or a sugar alcohol. The former discloses that the preparation has no pleasant feeling in a mouth when administered when it is prepared by using a low-substituted hydroxypropyl cellulose having a loose bulk density of less than 0.35 g/ml or a tapped bulk density of less than 0.60 g/ml, and on the other hand, the latter discloses that the preparation has no pleasant feeling in a mouth when administered when it is prepared by using a low-substituted hydroxypropyl cellulose having a loose bulk density of less than 0.40 g/ml or a tapped bulk density of less than 0.60 g/ml.

With regard to an external lubricating compression method, it has recently been published to provide a rotary type powder compressing machine which can make tableting with continuously supplying a powdered lubricant.

For example, JP-A-2001-205493 discloses a rotary type power compressing machine equipped with a powdered lubricant spraying means for spraying a powdered lubricant onto the upper punches and the lower punches to prevent the sticking with powdered materials on the punches and the dies, and an air flow supplying system to prevent scattering of the surplus amount of the powdered lubricant which do not adhere onto the upper punches and the lower punches. Further JP-A-2001-293599 discloses a rotary type powder compressing machine equipped with a powdered lubricant supplying means for spraying a powered lubricant onto the upper and lower punches as mentioned above, a flow detector for detecting the amount of the powdered lubricant flowed out, a flow detector for detecting the unused, surplus amount of the powdered lubricant, and a controller for controlling the supplier of the powdered lubricant by calculating the amount to be flowed on the basis of the above detected amounts. However, these publications do not mention about any specific tablet formulation to be produced by the above rotary type powder compressing machine.

Besides, it is also described in Hitoshi KUSUNOKI, "External Lubricating System for Tabletting Machine", Journal of Japan Society of Pharmaceutical Machinery and Engineering, Vol. 10, No. 2 (2001), p. 60-66, issued by Japan Society of Pharmaceutical Machinery and Engineering that tablets containing no lubricant inside thereof can be produced with preventing from adhesion of powder onto the mold by an external lubricant spraying system wherein the lubricant is directly spraying onto the upper and lower punches and the dies so as to form a coating of the lubricant on the surface of the molded powder. However, this literature does still not disclose any specific tablet formulation to be produced by the external lubricant spraying system.

### DISCLOSURE OF INVENTION

It has been desired to produce, in a large scale and at a low cost, the tablets which exhibit a sufficient hardness so that the tablets can be handled without difficulty during from the tableting process until administration, a pleasant feeling in a mouth when administered and a good disintegrability in the oral cavity. The present inventors have intensively studied a method for producing an intrabuccally rapidly disintegrating tablet having the desired excellent rapid disintegrability and excellent economic merit, in a large scale, at a low cost and without using any especial ingredient. As a result, it has been found that by using an external lubricating technique to coat previously a lubricant (hereinafter, occasionally referred to as "external lubricating compression method"), especially by using a rotary type powder compressing machine equipped with an external lubricant supplying means which is suitable for applying a lubricant onto the punches and the dies, which is effected by spraying a dispersion of the lubricant in a constant air flow onto the punches and the dies in the tableting step, it is possible to produce, in large amount and in the desirable economical way, the desired small-sized, intrabuccally rapidly disintegrating tablets having a sufficient hardness, a pleasant feeling in a mouth when administered, and a good disintegrability in the oral cavity, and that even if D-mannitol having a comparatively large particle diameter, i.e. an average particle diameter of 31 µm to 80 µm is used, the desired tablets can be obtained by combining said D-mannitol with an active ingredient, a disintegrant such as a low-substituted hydroxypropyl cellulose, and stearic acid or a metallic stearate as a lubricant in an amount of not more than 0.5 % by weight.

The present invention provides an intrabuccally rapidly disintegrating tablet, which comprises D-mannitol having an average particle diameter of 31 µm to 80 µm, an active ingredient, a disintegrant, and 0.01 % by weight to 0.5 % by weight of stearic acid or a metallic stearate, and provides also a method for producing an intrabuccally rapidly disintegrating tablet containing stearic acid or a metallic stearate in an amount of 0.01 % by weight to 0.5 % by weight by tableting consecutively a powder containing D-mannitol having an average particle diameter of 31 µm to 80 µm, an active ingredient and a disintegrant (a composition for external lubricating compression) by the external lubricating compression method, which comprises the steps of previously spraying stearic acid or a metallic stearate to adhere onto the punches and the dies of a tableting machine, compressing the powder with the tableting machine, and recovering the unused, surplus stearic acid or a metallic stearate which do not adhere onto the punches and the dies, these procedures and tableting being consecutively repeated. The present invention further provides an intrabuccally rapidly disintegrating tablet produced by said method, and also provides a composition for external lubricating compression which comprises D-mannitol having an average particle diameter of 31 µm to 80 µm, an active ingredient and a disintegrant.

The terms used in the present description are explained below.

The "external lubricating compression method" generally means a method or a technique of molding powdered materials in a compressing molding apparatus without adding a lubricant such as magnesium stearate into the powdered materials, which comprises the steps of spraying or applying a lubricant onto the inner wall of the die and onto the contact surface of the punch by using optionally a suitable apparatus, filling the die with the powdered materials, and then compressing the materials, as disclosed in JP-B-41-11273. In the present invention, it is preferable to disperse a lubricant into the air flow at a constant air flow rate in order to obtain tablets having a more uniform quality. That is, by dispersing a lubricant into air flow at a constant air flow rate and spraying it, the lubricant can adhere to the inner wall of the die and to the contact surface of the punch in an almost uniform amount, as a result, the amount of lubricant to be contained in the tablets can almost be uniform. Therefore, stearic acid or a metallic stearate as a lubricant in the present invention can be usually localized almost on the surface of a tablet. However, depending on the kind of a compression machine equipped with an apparatus for supplying a lubricant, all of the lubricant sprayed cannot occasionally be adhered onto the punches and inner wall of the dies. In such a case, a part of the lubricant may be contaminated into the composition for external lubricating compression when the composition is supplied, and as a result, a part of the lubricant may be contained in the internal part of the tablets. However, it may usually not cause any bad effects on the properties of tablets as long as the lubricant contained in the internal part of the tablets is in a small amount. Therefore, these tablets are also inclusive in the intrabuccally rapidly disintegrating tablet of the present invention. The constant air flow rate for dispersing a lubricant is usually 5 L/min. (Normal) to 30 L/min. (Normal), preferably 8 L/min. (Normal) to 15 L/min. (Normal) [L/min. (Normal) is a unit for expressing an amount of air, and expresses the gas flow amount reduced under the conditions of 1 atm at 0°C]. Besides, the surplus lubricant which is sprayed but not used in the tableting can be recovered by a decompression system. The means and apparatuses for carrying out the above external lubricating compression method used in the present invention are, for example, a means or an apparatus for supplying externally a lubricant and a means for recovering the external lubricant as disclosed in above JP-A-2001-205493 and JP-A-2001-293599. The JP-A-2001-293599 publication discloses to produce tablets by carrying out repeatedly and consecutively the compression with a rotary type compressing machine equipped with a powdered lubricant supplying means.

Besides, it is also described in detail in Hitoshi KUSUNOKI, "External Lubricating System for Tabletting Machine", Journal of Japan Society of Pharmaceutical Machinery and Engineering, Vol. 10, No. 2 (2001), p. 60-66, issued by Japan Society of Pharmaceutical Machinery and Engineering as to a means and apparatus which is usable for carrying out the external lubricating compression method of the present invention, that is, an apparatus which has a spray nozzle for spraying a lubricant directly onto the upper and lower punches and dies so that a small amount of lubricant can be uniformly supplied to them. Said Hitoshi KUSUNOKI's literature discloses that the constant air flow rate for the dispersion of lubricant during the external lubricating compression is 10 L/min. (Normal) at the minimum. However, according to the method of the present invention, the lubricant can well be dispersed at an air flow rate of 5 L/min. (Normal) to 30 L/min. (Normal) and the tableting can consecutively be done with less scattering of the powders for tableting within the tableting machine. Preferred air flow rate is in the range of 8 L/min. (Normal) to 15 L/min. (Normal). When the air flow rate is more than 30 L/min. (Normal), the powders for tableting scatter in the tableting machine which badly affects on the consecutive tableting.

In the present specification, when simply expressed with "% by weight", it means % by weight based on the whole weight of a tablet, considering the whole weight of a tablet as 100 % by weight.

D-mannitol used in the present invention is a kind of sugar alcohols, and the average particle diameter thereof can be measured, for example, by a laser diffraction particle diameter analyzer "HELOS & RODOS" manufactured by Sympatec Inc. The average particle diameter of D-mannitol is usually 31 µm to 80 µm, preferably 40 µm to 70 µm. The amount of D-mannitol to be contained may vary according to the amounts of other pharmaceutical components such as excipients mentioned below. However, when other pharmaceutical components are not added thereto, D-mannitol is contained preferably in an amount of 39.5 % by weight to 99 % by weight, more preferably 80 % by weight to 98 % by weight.

As the disintegrant, a low-substituted hydroxypropyl cellulose, crystalline cellulose, carmellose calcium, and croscarmellose sodium are exemplified, preferably a low-substituted hydroxypropyl cellulose.

The low-substituted hydroxypropyl cellulose used in the present invention has usually a hydroxypropoxyl content of 5.0 % by weight to 16.0 % by weight and preferably has a loose bulk density of 0.20 g/ml to less than 0.40 g/ml, and particularly preferably the low-substituted hydroxypropyl cellulose has a loose bulk density of 0.20 g/ml to less than 0.40 g/ml, an average particle diameter (measured in the same manner as used for D-mannitol) of 30 µm to less than 60 µm, and a hydroxypropoxyl content of 10.0 % by weight to 16.0 % by weight. Preferable example of the low-substituted hydroxypropyl cellulose is LH-21 manufactured by Shin-Etsu Chemical Co., Ltd. The low-substituted hydroxypropyl cellulose is contained preferably in an amount of 1 % by weight to 10 % by weight, more preferably 2 % by weight to 7 % by weight. The loose bulk density can be measured, for example, by the method disclosed in JP-A-2001-322927 and JP-A-2001-328948.

The metallic stearate includes, for example, magnesium stearate, calcium stearate, and aluminum stearate, and magnesium stearate is preferable. The average particle diameter of the metallic stearate (which is measured in the same manner as used for D-mannitol) is 0.5 µm to 50 µm, preferably 1 µm to 30 µm. Besides, the metallic stearate is usually contained in an amount of 0.01 % by weight to 0.5 % by weight, preferably 0.01 % by weight to 0.3 % by weight.

Any medicament which can usually be formulated into a tablet can be added in the present tablet, and the medicament used in the present invention (occasionally referred to as "active ingredient" in the present specification) may be any form such as solid, crystal, oil or solution, and includes one or more ingredients such as nourishing and cordial agents, antipyretic-analgesic-antiinflammatory drugs, psychotropics, antianxiety drugs, antidepressants, hypnotic-sedative drugs, spasmolytics, central nervous system drugs, brain metabolism ameliorating agents, brain circulation ameliorating agents, antiepileptics, sympathomimetics, drugs for digestive organ, gastrointestinal agents, antacid, antiulcer agents, antitussive-expectorants, antiemetics, respiratory accelerators, bronchodilators, antiallergic drugs, dental buccal drugs, antihistamines, cardiotonics, antiarrhythmic drugs, diuretics, antihypertensive agents, vasoconstrictors, coronary vasodilators, peripheral vasodilators, antihyperlipidemic agents, cholagogues, antibiotics, chemotherapeutic drugs, antidiabetic agents, drugs for osteoporosis, antirheumatoid agents, skeletal muscle relaxants, antispasmodics, hormones, alkaloid narcotics, sulfa drugs, arthrifuges, blood coagulation inhibitors, antitumor agents, etc.

Specific examples of the active ingredient are acetaminophen and ketoprofen which are antiphlogistic-analgesic drugs, diphenhydramine and ebastine which are antiallergic drugs, erythromycin and sparfloxacin which are antibiotics and antibacterial drugs, alacepril which is antihypertensive-ACE Inhibitor, mosapride citrate which is gastrointestinal motility accelerator, loperamide hydrochloride and other salts thereof which are antidiarrheal drugs, haloperidol which is antipsychotic drug, nitrazepam which is antianxiety drug, imipramine and moclobemide which are antidepressants, phenytoin, zonisamide, and sodium valproate which are antiepileptic drugs, farnesol which is antiulcer agents, ephedrine and other salts thereof which are bronchodilators, codeine phosphate which is antitussive drug, morphine acid salts such as morphine hydrochloride and morphine sulfate which are narcotic analgesics, brotizolam which is hypnotic, verapamil and diltiazem which are antianginal drugs, gliclazide which is hypoglycemic drug, etc.

The active ingredient is usually contained in an amount of 0.01 % by weight to 70 % by weight, preferably 0.1 % by weight to 50 % by weight, more preferably 1 % by weight to 10 % by weight.

The average particle diameter of the active ingredient (measured in the same manner as used for D-mannitol) is usually not more than 500 µm, preferably 0.1 µm - 100 µm, more preferably 1 µm - 20 µm.

The intrabuccally rapidly disintegrating tablet of the present invention may optionally contain other components, such as excipients, stabilizers, surfactants, binders, other lubricants than stearic acid and a metallic stearate, fluidization agents, coloring agents, sweetening agents, flavors, etc. unless they give bad effect on the tablet.

Suitable examples of the excipients are lactose, starch, sucrose, erythritol, trehalose, calcium hydrogenphosphate anhydrous, and calcium sulfate.

Suitable examples of the stabilizers are disodium edetate, tocopherol, etc.

Suitable examples of the surfactants are sodium lauryl sulfate, polysorbates, hydrogenated oil.

Suitable examples of the binders are gum arabic, starch, hydroxypropyl cellulose, hydroxypropyl methylcellulose, polyvinylalcohol, pullulan, gelatin, ethyl cellulose, methyl cellulose, carmellose sodium, dextrin, and polyvinylpyrrolidone.

Suitable examples of the lubricants other than stearic acid and a metallic stearate are sucrose fatty acid esters, talc, hydrogenated oil, and macrogol.

Suitable examples of the fluidization agents are light anhydrous silicic acid, etc.

Suitable examples of the coloring agents are food dye, iron sesquioxide, carmine, etc.

Suitable examples of the sweetening agents are thaumatin, aspartame, stevia, saccharin sodium, etc., preferably thaumatin.

Suitable examples of the flavors are various fruit-flavors (e.g. strawberry flavor), yogurt, mint, menthol, etc.

Thus, the preferable intrabuccally rapidly disintegrating tablet of the present invention comprises D-mannitol having an average particle diameter of 31 µm to 80 µm, an active ingredient in an amount of 0.1 % by weight to 50 % by weight, a low-substituted hydroxypropyl cellulose in an amount of 1 % by weight to 10 % by weight, and stearic acid or a metallic stearate in an amount of 0.01 % by weight to 0.5 % by weight, wherein stearic acid or a metallic stearate is localized almost on the surface of the tablet.

In more preferable intrabuccally rapidly disintegrating tablet in the above formulation, the low-substituted hydroxypropyl cellulose has a loose bulk density of 0.20 g/ml to less than 0.40 g/ml, an average particle diameter of 30 µm to less than 60 µm, and a hydroxypropoxyl content of 10.0 % by weight to 16.0 % by weight, and the metallic stearate used as the lubricant is magnesium stearate.

As the most preferable example of the above intrabuccally rapidly disintegrating tablet, it contains 1 % by weight to 10 % by weight of ebastine having an average particle diameter of 1 µm to 20 µm as an active ingredient and further contains a sweetening agent, especially thaumatin.

The method of the present invention is more specifically explained below.

The present invention provides a method for producing an intrabuccally rapidly disintegrating tablet by consecutively tableting a powder material comprising D-mannitol having an average particle diameter of 31 µm to 80 µm, an active ingredient, a disintegrant, and optionally other ingredients with a tableting machine equipped with an external lubricant supplying apparatus and an external lubricant recovering apparatus, which is characterized by previously consecutively spraying stearic acid or a metallic stearate as a lubricant to adhere onto the punches and the dies of the tableting machine and consecutively recovering the unadherent surplus of stearic acid or a metallic stearate, which is sprayed but not used in the tableting, so that the content of stearic acid or a metallic stearate in the obtained tablets becomes in an amount of 0.01 % by weight to 0.5 % by weight. It is preferable to spray the lubricant onto the punches and the dies while dispersing it into a constant air flow. The starting powder material to be subjected to the tableting, i.e. a composition for external lubricating compression can be obtained by mixing D-mannitol having an average particle diameter of 31 µm to 80 µm and a disintegrant with a blender machine, or alternatively by spraying water or an aqueous solution or dispersion containing optionally a coloring agent and a sweetening agent to the mixture of D-mannitol and disintegrant, granulating the mixture by fluid bed granulation or wet high-shear granulation, and then drying the granules. An active ingredient can be added anytime before the tableting process. The powder material containing an active ingredient (a composition for external lubricating compression) can be compressed by means of a rotary type powder compression machine equipped with an external lubricant supplying apparatus and an external lubricant recovering apparatus as described in Hitoshi KUSUNOKI, "External Lubricating System for Tabletting Machine", Journal of Japan Society of Pharmaceutical Machinery and Engineering, Vol. 10, No. 2 (2001), p.60-66, issued by Japan Society of Pharmaceutical Machinery and Engineering, for example a rotary type powder compression machine as mentioned in JP-A-2001-205493 and JP-A-2001-293599, and thereby the intrabuccally rapidly disintegrating tablet of the present invention is obtained. The pressure for compression is usually in the range of 50 MPa/cm² to 250 MPa/cm², preferably 80 MPa/cm² to 200 MPa/cm².

In the intrabuccally disintegrating tablet of the present invention thus obtained, the lubricant thereof, i.e. a metallic stearate or stearic acid is localized almost on the surface of a tablet and hence, it does not show the disadvantages shown by the conventional tablets, such as the delayed disintegration caused by the inevitable internal existence of a lubricant such as a metallic stearate, but it exhibits advantageously rapid disintegrability in oral cavity and adequate tensile strength. The disintegration time of the intrabuccally rapidly disintegrating tablet of the present invention is usually 5 seconds to 30 seconds, preferably 5 seconds to 20 seconds, and the tensile strength thereof is usually 100 N/cm² to 300 N/cm², preferably 120 N/cm² to 200 N/cm².

The present invention has an advantage which is a wide range of formulating design because the method of external lubricating compression makes the porosity of the tablet small and enables to produce a small tablet, which is different from a current intrabuccally rapidly disintegrating tablets.

### BEST MODE FOR CARRYING OUT THE INVENTION

The present invention is illustrated in more detail by the following Examples and Comparative examples, but it should not be construed to be limited thereto. In the following Examples, the surplus of magnesium stearate sprayed but not used was consecutively recovered by the recovering machine constituted with a dust collector when tablets were produced with a rotary type tableting machine (Kikusui Seisakusho Ltd., Type Collect 19 K) using an external lubricant spraying system equipped with an external lubricant supplying apparatus and a recovering apparatus (hereinafter, referred to as "external lubricant spraying system") (Kikusui Seisakusho Ltd., ELS-P1). Besides, as to the tablets produced in the following examples using the external lubricant spraying system, the amount of magnesium stearate per whole weight of a tablet was determined by an atomic emission spectrometry via measuring the amount of magnesium in the tablet produced.

### Example 1

0.5 g of ebastine (average particle diameter : 5 µm), 1 g of a low-substituted hydroxypropyl cellulose (LH-21, manufactured by Shin-Etsu Chemical Co., Ltd., average particle diameter : 37 µm, loose bulk density : 0.34 g/ml, hydroxypropoxyl content : 10.9 % by weight), 18.49 g of D-mannitol (MANNITOL 60, manufactured by ROQUETTE, average particle diameter : 60 µm), and 0.01 g of thaumatin (Sansuito T, manufactured by San-Ei Gen F. F. I., Inc.) were mixed in a polyethylene bag. And then the mixture was compressed under a pressure of about 150 MPa/cm² using a tableting machine (Kikusui Seisakusho Ltd., Type 2B) wherein magnesium stearate (manufactured by TAIHEI CHEMICALS LIMITED) was coated on the punches and the dies in an amount of about 0.3 % by weight based on whole weight of a tablet to provide tablets each weighing 200 mg and having 8 mm in diameter.

### Example 2

Ten g of ebastine (average particle diameter : 5 µm), 10 g of a low-substituted hydroxypropyl cellulose (LH-21, manufactured by Shin-Etsu Chemical Co., Ltd., average particle diameter : 37 µm, loose bulk density : 0.34 g/ml, hydroxypropoxyl content : 10.9 % by weight), 179 g of D-mannitol (MANNITOL 60, manufactured by ROQUETTE, average particle diameter : 60 µm), and 1 g of light anhydrous silicic acid (Aerosil, manufactured by Nippon Aerosil) were mixed in a polyethylene bag. The mixture was compressed under a pressure of about 150 MPa/cm² by using a rotary tableting machine (Kikusui Seisakusho Ltd., Type Collect 19 K), wherein the punches and the dies in the tableting machine were coated with magnesium stearate (manufactured by TAIHEI CHEMICALS LIMITED) by supplying it with a constant air flow rate of 10 L/min. (Normal) at a supplying rate of about 10 g/h with an external lubricant spraying system (Kikusui Seisakusho Ltd., ELS-P1). Thus, the desired tablets (weighing 100 mg and having 6.5 mm in diameter) were produced. The tablets had magnesium stearate content of about 0.1 % by weight based on whole weight of a tablet.

### Example 3

A hundred g of ebastine (average particle diameter : 5 µm), 100 g of a low-substituted hydroxypropyl cellulose (LH-21, manufactured by Shin-Etsu Chemical Co., Ltd., average particle diameter : 37 µm, loose bulk density : 0.34 g/ml, hydroxypropoxyl content : 10.9 % by weight), and 1599 g of D-mannitol (MANNITOL 60, manufactured by ROQUETTE, average particle diameter : 60 µm) were granulated in a fluid bed granulating and drying machine (Freund Industrial Co., Ltd., FLO-2) while spraying 2000 g of an aqueous solution containing 0.05 % (w/w) of thaumatin (Sansuito T, manufactured by San-Ei Gen F. F. I., Inc.) and 10 % (w/w) of D-mannitol, and then they were dried. The mixture was compressed under a pressure of about 150 MPa/cm² by using a rotary tableting machine (Kikusui Seisakusho Ltd., Type Collect 19 K), wherein the punches and the dies in the tableting machine were coated with magnesium stearate (manufactured by TAIHEI CHEMICALS LIMITED) by supplying it with a constant air flow rate of 8 L/min. (Normal) at a supplying rate of about 15 g/h with an external lubricant spraying system (Kikusui Seisakusho Ltd., ELS-P1). Thus, the desired tablets (weighing 100 mg and having 6.5 mm in diameter) were produced. The tablets had magnesium stearate content of about 0.2 % by weight based on whole weight of a tablet.

### Example 4

Fifty g of ebastine (average particle diameter : 5 µm), 50 g of a low-substituted hydroxypropyl cellulose (LH-21, manufactured by Shin-Etsu Chemical Co., Ltd., average particle diameter : 37 µm, loose bulk density : 0.34 g/ml, hydroxypropoxyl content : 10.9 % by weight), 898.5 g of D-mannitol (MANNITOL 60, manufactured by ROQUETTE, average particle diameter : 60 µm) and 0.5 g of light anhydrous silicic acid (Aerosil, manufactured by Nippon Aerosil) were granulated in a fluid bed granulating and drying machine (Freund Industrial Co.,Ltd., FLO-2) while spraying 500 g of an aqueous solution containing 0.20 % (w/w) of thaumatin (Sansuito T, manufactured by San-Ei Gen F. F. I., Inc.), and then they were dried. They were compressed under a pressure of about 150 MPa/cm² using a tableting machine (Kikusui Seisakusho Ltd., Type 2B) wherein magnesium stearate (manufactured by TAIHEI CHEMICALS LIMITED) was coated on the punches and the dies in an amount of about 0.1 % by weight based on whole weight of a tablet to provide tablets each weighing 200 mg and having 8 mm in diameter.

### Example 5

0.25 g of brotizolam, 10 g of a low-substituted hydroxypropyl cellulose (LH-21, manufactured by Shin-Etsu Chemical Co., Ltd., average particle diameter : 37 µm, loose bulk density : 0.34 g/ml, hydroxypropoxyl content : 10.9 % by weight), and 189.55 g of D-mannitol (MANNITOL 60, manufactured by ROQUETTE, average particle diameter : 60 µm) were mixed in a polyethylene bag. And then they were compressed under a pressure of about 150 MPa/cm² using a tableting machine (Kikusui Seisakusho Ltd., Type 2B) wherein magnesium stearate (manufactured by TAIHEI CHEMICALS LIMITED) was coated on the punches and the dies in an amount of about 0.1 % by weight based on whole weight of a tablet to provide tablets each weighing 200 mg and having 8 mm in diameter.

### Example 6

A hundred g of a low-substituted hydroxypropyl cellulose (LH-21, manufactured by Shin-Etsu Chemical Co., Ltd., average particle diameter : 37 µm, loose bulk density : 0.34 g/ml, hydroxypropoxyl content : 10.9 % by weight) and 1795 g of D-mannitol (MANNITOL 60, manufactured by ROQUETTE, average particle diameter : 60 µm) were granulated in a fluid bed granulating and drying machine (Freund Industrial Co.,Ltd., FLO-2) while spraying 400 g of an aqueous solution containing 0.5 % (w/w) of thaumatin (Sansuito T, manufactured by San-Ei Gen F. F. I., Inc.) which was suspended with 0.5 g of carmine (Carmine "San-Ei", manufactured by San-Ei Gen F. F. I., Inc.), and then were dried. 1703.75 g of the resulting granules, 90 g of ebastine (average particle diameter : 5 µm), 4.9 g of light anhydrous silicic acid (Aerosil, manufactured by Nippon Aerosil) and 1.35 g of strawberry flavoring (Sanfix Natural Strawberry, manufactured by San-Ei Gen F. F. I., Inc.) were mixed with a V-blender (manufactured by Fuji Paudal Co., Ltd., VM-10), and the mixture was compressed under a pressure of about 150 MPa/cm² by using a rotary type tableting machine (Kikusui Seisakusho Ltd., Type Collect 19 K), wherein the punches and the dies in the tableting machine were coated with magnesium stearate (manufactured by TAIHEI CHEMICALS LIMITED) by supplying it with a constant air flow rate of 13 L/min. (Normal) at a supplying rate of about 10 g/h with an external lubricant spraying system (Kikusui Seisakusho Ltd., ELS-P1). Thus, the desired tablets (weighing 200 mg and having 8 mm in diameter) were produced. The tablets had magnesium stearate content of about 0.1 % by weight based on whole weight of a tablet.

### Example 7

Fifty g of a low-substituted hydroxypropyl cellulose (LH-21, manufactured by Shin-Etsu Chemical Co., Ltd., average particle diameter : 37 µm, loose bulk density : 0.34 g/ml, hydroxypropoxyl content : 10.9 % by weight), 896 g of D-mannitol (MANNITOL 60, manufactured by ROQUETTE, average particle diameter : 60 µm), 2 g of light anhydrous silicic acid (Aerosil, manufactured by Nippon Aerosil) and 50 g of ebastine (average particle diameter : 5 µm) were granulated in a wet high-shear granulating machine (Powrex Corp., FM-VG-05) while spraying 130 g of an aqueous solution containing 0.58 % (w/w) of thaumatin (Sansuito T, manufactured by San-Ei Gen F. F. I., Inc.), and then dried with a tray type dryer. The resulting granules were sifted by means of a 22 mesh sieve (opening, 710 µm). The sieved granules were compressed under a pressure of about 150 MPa/cm² by using a rotary type tableting machine (Kikusui Seisakusho Ltd., Type Collect 19 K), wherein the punches and the dies of the tableting machine were coated with magnesium stearate (TAIHEI CHEMICALS LIMITED) by supplying it with a constant air flow rate of 10 L/min. (Normal) at a supplying rate of about 15 g/h with an external lubricant spraying system (Kikusui Seisakusho Ltd., ELS-P1). Thus, the desired tablets (weighing 200 mg and having 8 mm in diameter) were produced. The tablets had magnesium stearate content of about 0.2 % by weight based on whole weight of a tablet.

### Comparative Example 1 : A case of using mannitol having a larger average particle diameter -

In the same manner as described in Example 1 except that D-mannitol (Mannit S, manufactured by Towa Kasei Co., Ltd., average particle diameter : 120 µm) was used, the tablets (weighing 200 mg and having 8 mm in diameter) were produced.

### Comparative Example 2 : A case of using a mannitol having a smaller average particle diameter -

In the same manner as described in Example 2 except that D-mannitol (manufactured by Towa Kasei Co., Ltd., average particle diameter : 16 µm) was used, the tablets (weighing 100 mg and having 6.5 mm in diameter) were produced.

### Comparative Example 3 : A case of the same formulation as in Example 6 except for lubricant, and further the method is not an external lubricating compression method -

A hundred g of a low-substituted hydroxypropyl cellulose (LH-21, manufactured by Shin-Etsu Chemical Co., Ltd., average particle diameter : 37 µm, loose bulk density : 0.34 g/ml, hydroxypropoxyl content : 10.9 % by weight) and 1795 g of D-mannitol (MANNITOL 60, manufactured by ROQUETTE, average particle diameter : 60 µm) were granulated in a fluid bed granulating and drying machine (Freund Industrial Co.,Ltd., FLO-2) while spraying 400 g of an aqueous solution of 0.5 % (w/w) of thaumatin (Sansuito T, manufactured by San-Ei Gen F. F. I., Inc.) which was suspended with 0.5 g of carmine (Carmine "San-Ei", manufactured by San-Ei Gen F. F. I., Inc.), and then were dried. 1703.75 g of the resulting granules, 90 g of ebastine (average particle diameter : 5 µm), 4.9 g of light anhydrous silicic acid (Aerosil, manufactured by Nippon Aerosil), and 1.35 g of strawberry flavor (Sanfix Natural Strawberry, manufactured by San-Ei Gen F. F. I., Inc.) were mixed with a V-blender (Fuji Paudal Co., Ltd., VM-10). 99 g of the resulting granules and 1 g of magnesium stearate (TAIHEI CHEMICALS LIMITED) were mixed in a polyethylene bag and then compressed under a pressure of about 150 MPa/cm² using a rotary tableting machine (Kikusui Seisakusho Ltd., Type 2B) to provide tablets (each weighing 200 mg and having 8 mm in diameter).

The disintegrating time in an oral cavity and the hardness of the tablets produced in Examples 1-7 and Comparative Examples 1-3 are shown in Table 1 and Table 2, respectively. It was evaluated that the tablets met the desired conditions of the present invention when they had a tensile strength of 120 N/cm² - 200 N/cm² and the disintegrating time of 5 sec. - 20 sec. under the conditions shown in the foot note of the following tables in the experiments of the tensile strength and the disintegrability, and furthermore they had pleasant feeling in a mouth when administered.

The tensile strength was calculated from the breaking load measured by a SCHLEUNIGER tablet hardness tester by the following equation.

Tensile Strength (N/cm²) = 2 X Breaking Load (N) / [π X Tablet Diameter (cm) X Tablet Thickness (cm)]

**Table 1**

| Example | Disintegrating Time*¹ (sec) | Tensile Strength (N/cm²) | Feeling in a Mouth when administered |
|---|---|---|---|
| 1 | 16 | 129 | good |
| 2 | 14 | 167 | good |
| 3 | 12 | 143 | good |
| 4 | 16 | 147 | good |
| 5 | 16 | 167 | good |
| 6 | 14 | 176 | good |
| 7 | 14 | 126 | good |

| | | | |
|---|---|---|---|
| *1 : Disintegrating Time : The time that a tablet is disintegrated under the conditions of a soft touch with tongue without biting a tablet in the oral cavity, by three healthy adult men as a subject. | | | |

**Table 2**

| Comparative Example | Disintegrating Time*¹(sec) | Tensile Strength (N/cm²) | Feeling in a Mouth when administered |
|---|---|---|---|
| 1 | 16 | 136 | sandy |
| 2 | ―*² | ―*² | ―*² |
| 3 | 25 | 88 | good |

| | | | |
|---|---|---|---|
| *1 : Disintegrating Time : The time that a tablet is disintegrated under the conditions of a soft touch with tongue without biting a tablet in the oral cavity, by three healthy adult men as a subject. | | | |
| *2 : Impossible to measure because it could not be tableted. | | | |

As shown in Table 1 and Table 2, both of the disintegrating time and the tensile strength of the tablets in Examples 1 - 7 of the present invention exhibited satisfactory results, but the tablet in Comparative Example 1 did not exhibit pleasant feeling in a mouth when administered, and the tablet in Comparative Example 3 showed lower tensile strength and the integrating time of 20 seconds or longer, and further the tablet in Comparative Example 2 had less fluidity powders and could not be tableted.

### INDUSTRIAL APPLICABILITY

As explained above, the present invention can provide the small-sized, intrabuccally rapidly disintegrating tablets having excellent properties, which exhibit a sufficient hardness not to disintegrate during usual handling, a good disintegrability in the oral cavity, and a pleasant feeling in a mouth when administered, by means of the external lubricating compression method which uses only D-mannitol having a comparatively large average particle diameter (31 µm - 80 µm) and conventional ingredients and localizes the lubricant which affects the hardness of the tablet and the disintegrablity, almost on the surface of a tablet. The method of the present invention enables to produce the above intrabuccally rapidly disintegrating tablet having excellent properties in a large scale and in simple way, by means of the external lubricating compression method wherein the lubricant is sprayed and recovered consecutively.

## Claims

1. An intrabuccally rapidly disintegrating tablet, which comprises D-mannitol having an average particle diameter of 31 µm to 80 µm, an active ingredient, a disintegrant, and stearic acid or a metallic stearate in an amount of 0.01 % by weight to 0.5 % by weight.

2. The intrabuccally rapidly disintegrating tablet according to claim 1, wherein the disintegrant is selected from a low-substituted hydroxypropyl cellulose, crystalline cellulose, carmellose calcium, and croscarmellose sodium.

3. The intrabuccally rapidly disintegrating tablet according to claim 2, which comprises D-mannitol having an average particle diameter of 31 µm to 80 µm, an active ingredient in an amount of 0.1 % by weight to 50 % by weight, a low-substituted hydroxypropyl cellulose in an amount of 1 % by weight to 10 % by weight, and stearic acid or a metallic stearate in an amount of 0.01 % by weight to 0.5 % by weight.

4. The intrabuccally rapidly disintegrating tablet according to claim 3, wherein the low-substituted hydroxypropyl cellulose has a loose bulk density of 0.20 g/ml to less than 0.40 g/ml.

5. The intrabuccally rapidly disintegrating tablet according to claim 3, wherein the low-substituted hydroxypropyl cellulose has a loose bulk density of 0.20 g/ml to less than 0.40 g/ml, an average particle diameter of 30 µm to less than 60 µm, and a hydroxypropoxyl content of 10.0 % by weight to 16.0 % by weight.

6. The intrabuccally rapidly disintegrating tablet according to any one of claims 1 to 5, wherein the active ingredient has an average particle diameter of 0.1 µm to 100 µm.

7. The intrabuccally rapidly disintegrating tablet according to any one of claims 1 to 6, wherein the active ingredient is ebastine.

8. The intrabuccally rapidly disintegrating tablet according to any one of claims 1 to 7, which further comprises a sweetening agent.

9. The intrabuccally rapidly disintegrating tablet according to any one of claims 1 to 8, wherein stearic acid or a metallic stearate is localized almost on the surface of the tablet.

10. A method for producing an intrabuccally rapidly disintegrating tablet containing stearic acid or a metallic stearate in an amount of 0.01 % by weight to 0.5 % by weight, which comprises tableting consecutively a powder containing D-mannitol having an average particle diameter of 31 µm to 80 µm, an active ingredient and a disintegrant (a composition for external lubricating compression) by an external lubricating compression method by the steps of previously spraying stearic acid or a metallic stearate to adhere onto punches and dies of a tableting machine, compressing the powder with the tableting machine, and recovering the surplus stearic acid or a metallic stearate which does not adhere onto the punches and dies in the compression step, these procedures and tableting being consecutively repeated.

11. A method for producing an intrabuccally rapidly disintegrating tablet containing stearic acid or a metallic stearate in an amount of 0.01 % by weight to 0.5 % by weight, which comprises tableting consecutively a powder containing D-mannitol having an average particle diameter of 31 µm to 80 µm, an active ingredient and a disintegrant (a composition for external lubricating compression) by an external lubricating compression method by the steps of previously spraying stearic acid or a metallic stearate to adhere onto punches and dies of a tableting machine which is effected by dispersing stearic acid or a metallic stearate into air flow at a constant flow rate, compressing the powder with the tableting machine, and recovering the surplus stearic acid or a metallic stearate which does not adhere onto the punches and dies in the compression step, these procedures and tableting being consecutively repeated.

12. A method according to claim 10 or 11, wherein the disintegrant is selected from a low-substituted hydroxypropyl cellulose, crystalline cellulose, carmellose calcium, and croscarmellose sodium.

13. A method according to claim 12, wherein the active ingredient is contained in the produced tablet in an amount of 0.1 % by weight to 50 % by weight and the low-substituted hydroxypropyl cellulose as the disintegrant is contained in the produced tablet in an amount of 1 % by weight to 10 % by weight.

14. A method according to claim 13, wherein the low-substituted hydroxypropyl cellulose has a loose bulk density of 0.20 g/ml to less than 0.40 g/ml.

15. A method according to claim 13, wherein the low-substituted hydroxypropyl cellulose has a loose bulk density of 0.20 g/ml to less than 0.40 g/ml, an average particle diameter of 30 µm to less than 60 µm, and a hydroxypropoxyl content of 10.0 % by weight to 16.0 % by weight.

16. A method according to any one of claims 10 to 15, wherein the active ingredient has an average particle diameter of 0.1 µm to 100 µm.

17. A method according to any one of claims 10 to 16, wherein the active ingredient is ebastine.

18. A method according to any one of claims 10 to 17, wherein the composition for external lubricating compression further comprises a sweetening agent.

19. A method according to any one of claims 10 to 18, wherein stearic acid or a metallic stearate is localized almost on the surface of the intrabuccally rapidly disintegrating tablet.

20. A method according to claim 11, wherein the constant air flow in the external lubricating compression system is at a rate of 5 L/min. (Normal) to 30 L/min. (Normal).

21. A method according to claim 11, wherein the constant air flow in the external lubricating compression system is at a rate of is 8 L/min. (Normal) to 15 L/min. (Normal).

22. An intrabuccally rapidly disintegrating tablet, which is produced by the method disclosed in claim 10 or 11.

23. A composition for external lubricating compression, which comprises D-mannitol having an average particle diameter of 31 µm to 80 µm, an active ingredient, and a disintegrant.

24. A composition for external lubricating compression according to claim 23, which contains as the disintegrant a low-substituted hydroxypropyl cellulose having a loose bulk density of 0.20 g/ml to less than 0.40 g/ml, an average particle diameter of 30 µm to less than 60 µm, and a hydroxypropoxyl content of 10.0 % by weight to 16.0 % by weight.
